# DEMANDE DE BREVET EUROPEEN

(11) **EP 0 736 770 A2**
(43) Date de publication de la demande: **09.10.1996**
(21) Numéro de dépôt: 96870042.7
(22) Date de dépôt: 01.04.1996
(51) Int. Cl.: G01N 33/531, G01N 33/68, G01N 33/543, G01N 33/547, C07K 16/44, A61K 39/385, C07K 16/42

(54) **Conjugué immunoréactif, procédé d'obtention de ce conjugué, anticorps dirigés contre ledit conjugué, composition pharmaceutique et dispositif de diagnostic les comprenant**

(30) Priorité: 05.04.1995 BE 9500316; 08.02.1996 BE 9600113
(71) Demandeur: ANDA BIOLOGICALS S.A., F-67067 Strasbourg Cédex (FR)
(72) Inventeur: Maes, Roland, 67190 Mutzig (FR)
(74) Mandataire: Van Malderen, Joelle

(57) **Abrégé**

La présente invention concerne un conjugué immunoréactif comprenant un ou plusieurs haptène(s) comportant un groupe sulfhydryl et choisi(s) parmi le groupe constitué par les acides aminés, les carbohydrates, les carbohydrates aminés, le phosphatidylinositol, la sphingosine et leurs dérivés nitrosylés, acylés et/ou acétylés, ledit haptène étant couplé à une protéine porteuse hydrosoluble d'un poids moléculaire supérieur à 8000 KD et/ou un support solide par un agent couplant susceptible de se fixer au groupement sulfhydryl dudit haptène.

## Description

### Objet de l'invention.

La présente invention concerne un conjugué immunoréactif, son procédé d'obtention, les anticorps dirigés contre ledit conjugué immunoréactif ainsi que les anticorps dirigés contre lesdits anticorps.

La présente invention concerne également le dispositif de diagnostic ainsi que la composition pharmaceutique comprenant ledit conjugué et/ou les dits anticorps, ainsi que leur utilisation.

### Arrière-plan technologique à la base de l'invention.

De nombreuses maladies ont une étiologie inconnue ou incertaine, où l'intervention d'une composante immunologique n'est pas à exclure. Ainsi, l'arthrite rhumatoïde ou le SIDA peuvent présenter une composante infectieuse (d'origine virale ou bactérienne) et/ou une composante immunologique, mais la relation entre ces deux manifestations pathologiques n'est pas encore élucidée.

De même, la migraine pourrait éventuellement être caractérisée par une composante immunologique semblable.

Cependant, il est à l'heure actuelle impossible de concevoir la possibilité d'existence d'auto-anticorps qui puissent avoir l'effet dévastateur que présente le syndrome d'immunodéficience acquise ou encore engendrer des céphalées intolérables ou des atrophies musculaires incapacitantes observées chez les patients souffrant de migraine et d'arthrite rhumatoïde. Il est également difficile de concevoir comment un agent infectieux peut être l'agent étiologique responsable induisant une telle synthèse d'auto-anticorps.

Le syndrome d'immunodéficience acquise (SIDA ou AIDS) est une conséquence de l'infection par les HIV; mais le syndrome apparaît avec un décalage de plusieurs mois ou même plusieurs années après le début de l'infection, sans qu'on sache quel est l'élément causal qui a provoqué ce passage de l'état latent vers le syndrome. Une composante auto-immune n'est pas envisagée, et d'ailleurs les méthodes nécessaires pour entreprendre une telle recherche n'existent pas.

L'arthrite rhumatoïde est expérimentalement provoquée chez l'animal (le rat) par injection de concentrés de mycobactéries sans qu'on sache quel rôle ces produits mycobactériens peuvent jouer dans le développement de cette arthrite expérimentale. Une composante auto-immune est soupçonnée dans l'arthrite rhumatoïde humaine parce que la gestation réduit ou supprime, pour la durée de la grossesse, l'évolution de la maladie. Cependant, les méthodes nécessaires pour entreprendre une telle recherche sur l'auto-immunité des malades ne sont pas disponibles et par ailleurs, on ne saurait que chercher.

La tuberculose est chronique chez beaucoup de malades, sans qu'on sache quel est le motif de cette persistance mycobactérienne, ni la cause du réveil de la maladie. La tuberculose s' accompagne d'une immunodépression chez les patients qui se présentent pour un traitement spécifique, mais les auto-anticorps potentiellement débilitants qui pourraient accompagner le phénomène d'immunosuppression ne sont ni soupçonnés ni, a fortiori, recherchés.

Il est également connu qu'une infection tuberculeuse s'accompagne de la production d'anticorps anti-glycolipides tels que l'anticorps anti-lipoarabinomannane, et que le mannose produit par des souches mycobactériennes virulentes permet d'échapper à la réaction immunitaire de l'hôte. Cependant, le mécanisme précis qui provoque ces réactions n'est pas connu.

En effet, l'existence d'auto-anticorps dirigés contre un sucre élémentaire tel que le mannose, l'acide acétyl-neuraminique ou la galactosamine n'est pas soupçonnée, et les techniques immunologiques nécessaires pour les mettre en évidence n'existent pas.

La migraine est d'étiologie inconnue, mais la douleur de la migraine est d'origine vasculaire. Aucun modèle de pathogenèse de la migraine ne se base sur une théorie auto-immune.

Cependant, la démonstration que les indolamines (tryptamine, sérotonine, méthoxytryptamine, etc.) présentes dans le cerveau peuvent être immunogènes après leur couplage à des molécules porteuses, que les anticorps qui sont induits artificiellement par vaccination peuvent être détectés (Antisera against the Indolealkylamines : tryptophan, 5-hydroxytryptophan, 5-hydroxytryptamine, 5-methoxytryptophan and 5-méthoxytryptamine tested by an ELISA method, M. Geffard et al., J. Neurochemistry, 44, 1221-1228 (1985)) et que la sérotonine est impliquée comme un médiateur de la migraine rend plausible l'hypothèse que des auto-anticorps anti-indoles peuvent être impliqués dans la migraine de tous ou du moins une partie des patients souffrant de maux de tête.

La possibilité d'induire expérimentalement des anticorps dirigés contre la sérotonine, la tryptamine et autres dérivés indoliques chez l'animal (par injection de conjugués de ces haptènes à de l'albumine et analyse subséquente du sérum par un test ELISA pour la détection des anticorps créés), a été décrite. Néanmoins, des tests diagnostiques suffisamment sensibles pour prouver leur hypothétique existence chez des patients souffrant de maux de tête, n'existent pas.

Pour arriver à obtenir artificiellement des anticorps dirigés contre de tels haptènes, il est nécessaire de préparer un conjugué constitué par lesdits haptènes couplés à une protéine porteuse (telle que la sérum-albumine bovine) ou un support solide (polymères, globules rouges, ...).

Pour obtenir un conjugué immunoréactif, (c'est-à-dire un conjugué contre lequel il est possible d'obtenir une synthèse importante d'anticorps), il est nécessaire d'arriver à obtenir le couplage de nombreux haptènes sur ladite protéine porteuse et/ou ledit support solide.

Cependant, à ce jour, il n'a pas été possible d'obtenir un tel conjugué qui soit immunoréactif et qui pourrait être utilisé dans un dispositif et un procédé de détection et/ou de quantification d'anticorps ou utilisé comme une composition pharmaceutique susceptible d'induire une réaction immunitaire humorale et/ou cellulaire.

En outre, le concept d'auto-anticorps dirigés contre des acides aminés, des carbohydrates et leurs dérivés, associés à des maladies auto-immunes, n'est pas d'actualité et représente une déviation intolérable du paradigme qui gouverne ce domaine de la recherche médicale.

### Buts de l'invention.

La présente invention vise à obtenir un nouveau conjugué comprenant un ou plusieurs haptènes couplés à une protéine porteuse, ledit conjugué étant immunoréactif.

La présente invention vise également à obtenir les anticorps dirigés contre ledit conjugué immunoréactif.

Un autre but de la présente invention est relatif à l'obtention d'un procédé de détection et/ou de quantification d'anticorps présents dans un fluide corporel d'un patient, comprenant l'utilisation du conjugué et/ou des anticorps selon l'invention.

Un but complémentaire de la présente invention concerne l'obtention d'un dispositif de diagnostic et/ou une composition pharmaceutique comprenant ledit conjugué et/ou lesdits anticorps selon la présente invention.

### Eléments caractéristiques de la présente invention.

La présente invention concerne un conjugué immunoréactif comprenant un ou plusieurs haptène(s) comportant un groupe sulfhydryl et choisis parmi le groupe constitué par les acides aminés, les dérivés d'acides aminés, les carbohydrates, les dérivés des carbohydrates, les carbohydrates aminés, les dérivés des carbohydrates aminés, le phosphatidylinositol, la sphingosine, les dérivés de sphingosine, la N-acétyl-D-sphingosine et/ou les dérivés de N-acétyl-D-sphingosine; ledit haptène étant couplé à une protéine porteuse hydrosoluble d'un poids moléculaire supérieur à 8000 KD et/ou à un support solide, par un agent couplant susceptible de se fixer au groupement sulfhydryl dudit haptène.

Selon la présente invention, on entend par acide aminé et dérivé d'acide aminé comportant un groupement sulfhydryl, un acide aminé tel que la cystéine ou un dérivé d'un tel acide aminé tel que la N-acétyl-L-cystéine, la cystéine-NO, la N-acétyl-L-cystéine-NO, la L-γ-glutamyl-L-cystéine, l'homocystéine, le mercapto-éthanol, la méthionine, la thiolhistidine, l'acétylméthionine, la S-adénosylhomocystéine, la N-acétyl-cystéine-glycine ou le glutathion, comprenant de manière native ledit groupement sulfhydryl.

Des dérivés d'acides aminés comportant un groupe sulfhydryl peuvent être également des acides aminés (en particulier les vingt acides aminés naturels, l'ornithine, la citrulline, ...) modifiés par des produits réactionnels qui substituent un groupement sulfhydryl (-SH) au groupement amine (-NH₂) et/ou au groupement hydroxyl (-OH) présent sur ces haptènes, selon des méthodes bien connues de l'Homme de l'Art.

Cette substitution peut par exemple être obtenue au moyen du réactif de Traut (2-iminothiolane-HCl) ou le N-succinimidyl S-acétylthioacétate. L'agent couplant utilisé dans le procédé selon l'invention est donc susceptible de se fixer au groupement sulfhydryl (-SH) dudit haptène.

Les dérivés d'acides aminés et de carbohydrates selon l'invention sont de préférence choisis parmi le groupe constitué par la glutamine, la N-acétyl-L-glutamine et la glutamine acylée, l'acide N-acétyl-glutamique et l'acide glutamique acylé, le diéthylnitrosamine, l'acétylméthionine, la N-acétyl-arginine et l'arginine acylée, la N-acétyl-ornithine et l'ornithine acylée, la carnitine, le N-éthyl-N-nitrosourée, le N-acétyl-L-homosérine et l'homosérine acylée, le N(ω)-nitro-L-arginine-méthyl-ester, la taurine, la nicotine, la N^{α}-acétyl-lysine, la N^{ε}-(carboxyméthyl) lysine, la N^{ε}-(carboxyméthyl) hydroxylysine, la N^{α}-acétyl-N^{ε}-(carboxyméthyl) lysine, les indolamines, en particulier le tryptophane, le 5-méthoxytryptophane, le N-acétyl-tryptophane et le tryptophane acylé, la 5-hydroxytryptamine (sérotonine), la 6-hydroxytryptamine, la 5-méthoxytryptamine, la 5,6-dihydroxytryptamine, l'acide 5-méthoxyindolacétique, l'acide 5-hydroxyindolacétique, la N-acétyl-5-méthoxytryptamine (mélatonine) le 5-méthoxytryptophol ou le 5-hydroxyptryptophol.

Les dérivés acétylés et acylés de ces substances qui ont été reconnus pour augmenter de façon significative l'immunogénéicité de ces molécules sont également compris dans ce groupe, de même que les molécules qui peuvent être immunologiquement plus réactives lorsqu'elles sont nitrosylées (par exemple les dérivés de mercapto-éthanol, la taurine, la méthionine, l'acétylméthionine, la nicotine et la thiolhistidine).

Les di- et tri-acides aminés tels que la cystéine-glycine, la N-acétyl-cystéine-glycine, la cystéine-glycine acylée, le glutathion, le N-acétyl-aspargyl-glutamate, la L-γ-glutamyl-L-cystéine sont également immunogènes.

Les dérivés acétylés et acylés de ces substances qui en augmentent l'immunoréactivité sont également compris dans ce groupe, de même que les molécules qui peuvent être immunologiquement plus réactives lorsqu'elles sont nitrosylées (par exemple les dérivés de glutathion, la L-γ-glutamyl-L-cystéine, et la N-acétyl-cystéine-glycine).

Les indolamines sont des métabolites présents dans les tissus mammaliens issus du métabolisme de la sérotonine et sont le résultat d'actions d'enzymes telles que la monoamine oxydase et la N-acétyltransférase.

L'haptène peut être également un dérivé de sphingosine, en particulier la D-sphingosine, le sphingosyl-galactopyranoside, la sphingosyl-phosphoryl-choline et la N-acétyl-D-sphingosine, modifié par la substitution d'un groupement sulfhydryl comme mentionné ci-dessus.

Un dernier exemple d'haptène modifié par l'addition d'un groupement sulfhydryl sont le phosphatidylinositol, les carbohydrates, les dérivés de carbohydrates, les carbohydrates aminés et les dérivés de carbohydrates aminés, en particulier le glucose, le mannose, le galactose, l'arabinose et l'acide muramique, ainsi que leurs dérivés aminés, la glucosamine, la galactosamine, la mannosamine et leurs dérivés acétylés, en particulier l'acide N-acétyl-neuraminique, l'acide N-acétyl-O-neuraminique, la N-acétyl-glucosamine, la N-acétyl-mannosamine, la N-acétyl-galactosamine, l'acide N-acétyl-muramique, la N,N-diacétyl-galactosamine, la N,N-diacétyl-glucosamine et les dérivés disaccharides tels que la N-N'-diacétyl-chitobiose, le 2,3-acétyl-tréhalose, le 2,3-diacétyl-tréhalose ou leurs substituts acylés tels que le stéarate, le palmitate, le phthiocéranate, l'hydroxyphthiocéranate, les disaccharides tels que le -α-(1→5) D-arabinofuranoside ou encore le 3,6-di-O-Me-β-D glucopyranosyl-(1→4)-2,3-di-O-Me-α-L-rhamnopyranose (immunospécifique pour la lèpre). Sont inclus dans ce groupe de carbohydrates et de leurs dérivés, le trisaccharide L-α-D-heptose (1→2) heptose (1→3) heptose et leurs dérivés acétylés immunogènes spécifiques de *Haemophilus*. Dans ce groupe sont aussi inclus le phosphatidyl-inositol mannoside (1-phosphatidyl-L-myo-inositol 2-O-α-D-mannopyranoside) et le dipeptide N-acétyl-muramyl-L-alanyl-D-isoglutamine et la S-adénosylhomocystéine.

Dans ce cas, la substitution des groupements hydroxyl par un groupement thiol peut être obtenue par le réactif de Traut en procédant à un pH égal ou supérieur à 10. De même, les carbohydrates et leurs dérivés peuvent être rendus réducteurs par l'addition de NaIO₄, cet agent oxydant libérant les aldéhydes des -oses et ces aldéhydes pouvant ensuite réagir avec les résidus aminés de la protéine porteuse.

Avantageusement, dans le conjugué immunoréactif selon la présente invention, la protéine porteuse est choisie parmi le groupe constitué par la gélatine, l'histone, l'albumine, de préférence l'albumine bovine délipidée, la polysérine ou la thyroglobuline.

Les supports solides sont des globules rouges, des dérivés polymériques (polystyrène, ... ou du latex) qui peuvent être couplés par des agents couplants aux haptènes ou au conjugué (comportant la protéine porteuse) selon l'invention. Préférentiellement, le support solide est constitué de polystyrène sensibilisé par la maléimide qui sera couplée aux haptènes et biomolécules par leur groupement sulfhydryl présent à l'état natif ou artificiellement créé (par exemple par le réactif de Traut). De tels supports solides sensibilisés par la maléimide fixant les groupements sulfhydryl sont distribués de manière commerciale (par exemple les plaques de microtitration "SULFHYDRYL BINDING ®" de COSTAR).

D'autres protéines porteuses ou supports solides bien connus de l'Homme de l'Art et susceptibles d'être couplés à l'haptène pour former le conjugué immunoréactif, ou au conjugué, selon la présente invention peuvent également être utilisés.

Avantageusement, dans le conjugué immunoréactif selon la présente invention, l'agent couplant est choisi parmi le groupe constitué par la parabenzoquinone, la p-toluquinone, la dichloroquinone, la chinhydrone ou la 2,6-dichlorobenzoquinone.

Cependant, d'autres agents couplants capables d'être couplés à une protéine et susceptibles de se fixer au groupement sulfhydryl (-SH) de l'haptène peuvent être utilisés.

De tels agents couplants peuvent être notamment le N,N'-O-phénylènedimaléimide ou le N-succinimidyl-3-(2-pyridyldithio)propionate, le succinimidyl-4-(N-maléimido-méthyl)cyclohexane-1-carboxylate ou son sulfo-dérivé.

La présente invention concerne également un procédé de préparation du conjugué immuno-réactif selon la présente invention dans lequel un ou plusieurs haptènes comportant un groupe sulfhydryl, sont couplés à une protéine porteuse hydrosoluble d'un poids moléculaire supérieur à 8000 KD et/ou à un support solide par un agent couplant (tel que décrit ci-dessus) susceptible de se fixer au groupement sulfhydryl dudit haptène.

Avantageusement, les groupements amines et/ou groupements hydroxyl de l'haptène ont été prélablement substitués par des groupements sulfhydryl tels que décrits ci-dessus.

Un autre aspect de la présente invention concerne l'anticorps (monoclonal ou polyclonal) et/ou des fragments (en particulier les portions Fab', Fab, F(ab')₂, ... de celui-ci) dirigé(s) contre le conjugué immunoréactif selon la présente invention.

En effet, la Demanderesse a démontré qu'il est possible d'obtenir un conjugué immunoréactif comprenant ledit haptène, c'est-à-dire un conjugué qui est caractérisé par une spécificité antigénique et/ou immunogénique (humorale et/ou cellulaire), et qu'il est possible d'obtenir une réaction croisée entre ledit conjugué et un anticorps dirigé contre ledit conjugué (cette réaction croisée ou liaison étant susceptible d'être détectée in vitro par les méthodes bien connues de l'Homme de l'Art).

La présente invention concerne également l'anticorps (monoclonal ou polyclonal) et/ou des fragments (en particulier les portions Fab', Fab, F(ab')₂, ... de celui-ci) dirigé(s) contre ledit anticorps.

Un autre aspect de la présente invention concerne le dispositif de diagnostic et de quantification, tel qu'une trousse de diagnostic ou une colonne de chromatographie comprenant le conjugué et/ou les anticorps (ou un fragment de ceux-ci) selon l'invention. Dans ledit dispositif de diagnostic et de quantification, le conjugué selon l'invention ou l'haptène peuvent être directement couplés sur un support solide tel que décrit précédemment.

Il est donc possible d'obtenir un procédé de détection et/ou de quantification d'anticorps présents dans un fluide corporel d'un patient tel qu'un sérum, lesdits anticorps étant spécifiques d'haptènes de faible poids moléculaire, le plus souvent indicatifs d'une pathologie comportant une réponse auto-immune. Selon ce procédé, on prélève un fluide corporel du patient, de préférence du sérum, on fait réagir les anticorps présents dans le fluide corporel avec le conjugué selon l'invention éventuellement fixé sur un support solide et on détecte et/ou quantifie la réaction croisée entre ledit conjugué et lesdits anticorps dirigés contre ledit conjugué.

On entend notamment par "quantification des anticorps", la détermination (par des méthodes connues de l'Homme de l'Art) du pourcentage d'immunoglobulines (de classes IgM, IgA, IgG, ...) présentes dans le fluide corporel.

La présente invention conerne également la composition pharmaceutique telle qu'un vaccin comprenant le conjugué et/ou les anticorps (ou des fragments de ceux-ci) selon l'invention et un véhicule pharmaceutique adéquat.

Des véhicules pharmaceutiques selon l'invention, adéquats pour l'administration orale, sont ceux connus pour une telle administration, par exemple sous forme de tablettes, enrobées ou non enrobées, de pilules, de capsules, de solutions ou de sirops.

D'autres véhicules pharmaceutiques adéquats sont utilisés selon le mode d'administration choisi (intraveineux, intramusculaire, ...).

En particulier, les véhicules pharmaceutiques adéquats et les adjuvants sont des vehicules et adjuvants communs, bien connus de l'Homme du Métier, qui permette d'induire ou de supprimer la réponse cellulaire et/ou humorale du système immunitaire.

Les compositions pharmaceutiques sont préparées selon des procédés généralement utilisés par les pharmaciens et peuvent comprendre des véhicules pharmaceutiquement adéquats, solides ou liquides, et non toxiques.

L'incorporation d'un milieu galénique peut être également envisagée.

Le pourcentage de produit actif (conjugué ou anticorps) / véhicule pharmaceutique peut varier selon de très larges gammes, uniquement limitées par la tolérance et le niveau d'acceptation du produit par le patient. Les limites sont en particulier déterminées par la fréquence d'administration.

Un dernier aspect de la présente invention concerne l'utilisation de la composition pharmaceutique selon l'invention pour la préparation d'un médicament destiné à la prévention et/ou au traitement des pathologies comportant une réponse auto-immune.

On entend par pathologie présentant une réponse. auto-immune, les maladies auto-immunes et/ou les maladies caractérisées par une infection entraînant une perturbation du système immunitaire.

De préférence, lesdites pathologies comportant une réponse auto-immune sont choisies parmi le groupe constitué par la sclérose en plaques, la sclérose amyotrophique latérale, l'hyperthyroïdie, l'arthrite, la cirrhose, les lupus, les syndromes antiphosphatides (accidents thrombotiques, dermatites bulleuses, ...), les syndromes néphrotiques, les accidents vasculaires cérébraux, la myasthénie, le cancer, la tuberculose et/ou le syndrome d'immuno-déficience acquis (AIDS).

Il est également possible d'inclure dans la définition d'une pathologie comportant une réponse auto-immune, la migraine.

### Description d'une forme d'exécution préférée de l'invention.

L'invention repose sur l'observation, connue depuis longtemps, que les agents infectieux induisant des désordres immunitaires ou des maladies telles que le syndrome d'immuno-déficience acquise ou la tuberculose, infectent les macrophages des personnes infectées et peuvent y résider longtemps, même si la mission principale de ces macrophages est l'élimination de ces agents infectieux.

Il a été également pris en considération que les radicaux libres oxydants, entre autres l'oxyde nitrique (NO), synthétisés dans les macrophages en première réponse à une infection, sont très nocifs pour les cellules elle-mêmes et que ces radicaux sont rapidement séquestrés par un acide aminé particulier (la cystéine).

La cystéine est le récepteur naturel de l'oxyde nitrique et forme de la cystéine-NO.

De façon inattendue, la Demanderesse a découvert qu'il est possible de former des immunoconjugués (ou conjugués immunoréactifs) de cystéine et de cystéine-NO en les couplant à une protéine porteuse telle que de la gélatine, une histone, de l'albumine ou toute autre protéine porteuse. Il est possible qu'un patient souffre d'affections diverses par le fait que des anticorps réagissent contre des immunoconjugués comprenant la cystéine-NO ou l'acide aminé naturel cystéine. Ceci a été observé dans un test ELISA où les puits étaient sensibilisés par le conjugué selon l'invention.

De manière inattendue, il est donc possible d'obtenir des conjugués immunoréactifs comprenant un ou plusieurs haptènes comportant un groupe sulfhydryl, ledit haptène étant couplé à une protéine porteuse par un agent couplant susceptible de se lier au groupement sulfhydryl (-SH) dudit haptène.

La fixation des haptènes est particulièrement efficace, c'est-à-dire qu'il est possible sur une seule protéine porteuse de coupler un grand nombre d'haptènes lorsqu'on utilise comme agent couplant la parabenzoquinone (PBQ).

La parabenzoquinone est une molécule qui fut proposée comme agent couplant pour la synthèse d'immunoconjugués constitués d'un enzyme couplé à un anticorps. L'action couplante de PBQ porte sur une variété de radicaux présents dans les protéines et polysaccharides (groupes aminés, sulfhydryl, -0H et -H) mais son usage a été rapidement abandonné en raison de l'inactivation de l'activité enzymatique et immunologique des conjugués obtenus (Coupling of enzymes to antibodies and antigens, Avrameas S. et al., Scand. J. Immunol. Vol 8, Suppl.7. 7-23 (1978)).

L'activation d'acides aminés, entre autre la cystéine, en vue de leur conjugaison avec des protéines ou autres molécules, est obtenue communément par l'intermédiaire :
1) du carbodiimide qui active la fonction carboxylique (-COOH) d'une molécule et réagit ensuite ou en même temps avec une fonction amine (-NH₂) d'une autre (ou de la même) molécule,
2) de la glutaraldéhyde qui réagit avec la fonction -NH₂ d'une molécule et une fonction -NH₂ d'une autre (ou de la même) molécule, ou encore
3) de l'anhydride succinique ou glutarique, maléique etc. qui attaque la fonction -NH₂ libre des molécules et la prolonge en une fonction carboxylique, laquelle est ensuite activée par la carbodiimide.

Si une activation des fonctions carboxyliques en phase organique est envisagée, ceci est possible par l'éthylchloroformate.

D'autres méthodes de couplage consistent en la substitution des amines primaires de deux molécules par un groupement liant -S-S. Ceci s'effectue généralement par un agent hétérobifonctionel dont une moitié succinimide s'implante au niveau des -NH₂ d'une molécule, habituellement les -NH₂ de la lysine, suivi de la libération du groupement thiol (-SH) constituant l'autre moitié de l'agent hétérobifonctionel couplant, par réduction (dithiothréitol ou mercaptoéthanol) d'une fonction S-S cachée, puis réaction du groupement thiol libéré dans cette molécule avec un groupe S-S caché d'une autre molecule activée par le même ou un autre agent hétérobifonctionel de même nature, possédant encore une moitié réactionelle S-S cachée non-réduite.

Le résultat de cette méthode de couplage, bien connue de l'Homme de l'Art, est la formation intermoléculaire d'un pont S-S couplant les deux molécules activées. L'agent hétérobifonctionel couplant préférentiellement utilisé pour ce type de couplage est le SPDP (N-Succinimidyl 3-(2-pyridyldithio)propionate. Si une des molécules destinées à la conjugaison possède déjà une fonction sulfhydryl (-SH), le processus d'activation et de couplage par le SPDP est simplifié de moitié.

Une grande variété de méthodes de couplage existe, soit comme variantes de celles décrites ici, soit comme systèmes différents (par exemple libération des fonctions aldéhydiques des sucres souvent présents dans les protéines glycosylées, au moyen de NaI0₄, puis réaction des aldéhydes libérés avec des fonctions amines des protéines) mais la plupart, sinon toutes les méthodes décrites, incorporent d'une façon ou d'une autre un groupement -NH₂, présent sur une molécule ou l'autre, dans le processus de la conjugaison. Le plus souvent, l'amine primaire modifiée appartient à la lysine. Même des protéines porteuses (hémocyanine, albumine) activées par une maléimide réactive avec des sulfhydryls, portent les fonctions maléimide uniquement greffées sur les résidus lysine de ces protéines porteuses.

Le mérite d'un couplage par l'intermédiaire de la parabenzoquinone réside d'abord dans le très grand nombre de sites réactionels greffés sur la protéine porteuse, non limités à la fonction epsilon-amine de la lysine ou aux groupes carboxyliques. Ensuite, la simplicité de la méthode permet que le groupement sulfhydryl (-SH) soit naturellement présent dans certaines protéines et acides aminés et dérivés aminés, soit introduit artificiellement dans les molécules destinées au couplage, n'est pas un groupement réactionel parmi d'autres mais bien un groupement réactionel préférentiel du PBQ. Enfin, ceci permet que l'attachement de la cystéine ou de la N-acétylcystéine au polymère porteur par l'intermédiaire du groupement thiol n'influe pas ou peu sur les qualités immunologiques de la cystéine ou N-acétylcystéine couplées. Les haptènes non soufrés, et leurs dérivés possesseurs d'un groupement -NH2, peuvent être utilisés tels quels et greffés au polymère porteur activé par la PBQ par l'intermédiaire de leur fonction amine, mais ils peuvent aussi avantageusement être modifiés par des produits réactionels simples qui substituent un groupement -SH au groupement -NH₂, selon des méthodes bien connues de l'Homme de l'Art, et être ensuite couplés avec une efficacité remarquable à la protéine porteuse par l'intermédiaire de la parabenzoquinone.

Le SPDP déjà décrit peut être utilisé pour remplacer un groupement -NH₂ d'haptène par un groupement -SH mais des molécules plus simples et d'utilisation plus simple, telles le réactif de Traut (2- Iminothiolane-HCl) ou encore le N-succinimidyl S-acétylthioacétate, peuvent également être utilisées, avec une grande simplicité dans la méthode de substitution. De même, outre la benzoquinone, on peut envisager d'utiliser la p-toluquinone, la dichloroquinone, la chinhydrone, la 2,6-dichlorobenzoquinone et autres dérivés de la benzoquinone pour remplir le rôle d'agent couplant rempli par la benzoquinone.

Différents conjugués immunoréactifs selon la présente invention ont été préparés en activant différentes protéines porteuses, telles que de l'albumine bovine délipidée, mais également de l'hémocyanine, des histones, de la polylysine ou de la polysérine, etc. par de la benzoquinone et en les couplant aux haptènes de l'invention.

Les acides aminés possèdent par définition un groupement aminé qui peut servir à leur greffage à des polymères porteurs mais dont la modification en groupement sulfhydryl est couramment pratiquée par l'Homme de l'Art. Les réactifs les plus communément utilisés sont le réactif de Traut (2-Iminothiolane-HCl) ou encore le N-succinimidyl S-acétylthloacétate (SATA). La réaction d'addition d'un groupement sulfhydryl à une amine primaire au moyen d'un de ces deux réactifs se réalise facilement à un pH supérieur à 7. Dans le cas du SATA, la terminaison sulfhydryl est protégée et peut être facilement libérée par traitement avec de l'hydroxylamine.

Il est possible de :
- soit activer l'haptène par de la benzoquinone, que cet acide soit modifié ou non par substitution d un groupement sulfhydryl à la place de l'amine primaire, puis mettre l'acide aminé activé en contact avec le polymère porteur,
- soit d'activer la protéine porteuse par de la benzoquinone pour ensuite y coupler l'haptène, que cet acide soit modifié ou non par substitution d'un groupement sulfhydryl à la place de l'amine primaire.

Préférentiellement, la substitution des amines primaires d'un haptène par un groupement sulfhydryl est pratiquée systématiquement. Les deux approches décrites, l'activation de l'haptène (acide aminé naturel ou dérivé d'acide aminé naturel) et l'activation de la protéine porteuse, se sont révélées également valables.

Préférentiellement, on activera la protéine porteuse, qui permet par son poids moléculaire élevé une élimination simple et rapide de la PBQ en excès, par filtration, chromatographie ou dialyse.

Une activation de la protéine porteuse par la benzoquinone a été décrite (Coupling of enzymes to antibodies and antigens, Avrameas S. et al., Scand. J. Immunol. Vol 8, Suppl.7. 7-23 (1978)). Ces conditions de couplage décrites sont inapplicables pour la conjugaison d'acides aminés ou de carbohydrates et leurs dérivés à une protéine porteuse.

De préférence, la protéine porteuse, telle que l'albumine sérique, humaine ou bovine, est solubilisée à une concentration de 10 mg / ml de solution dans une solution aqueuse tamponnée à un pH acide, préférablement aux environs de pH 6. Le PBQ solubilisé à raison de 5 à 10 mg / ml dans un liquide organique (alcool, acétone ou autre solvant miscible à l'eau) est introduit dans la solution de protéine, en proportion variable selon les besoins de la synthèse. Une quantité, en poids, de PBQ égale au 1/50^{ème} à 1/200^{ème} du poids de protéine est préférée lorsque la protéine est de l'albumine. La réaction a lieu à une température qui peut varier de 0 à 37 ^{o}C ou même plus, à l'obscurité, durant un minimum de 1 heure mais qui peut être prolongée aussi longtemps que désiré. Normalement la réaction est poursuivie durant 2 heures à température ambiante.

Après activation de la protéine porteuse par le PBQ ou par un autre constituant de la famille des quinones, le pH de la solution est rendu basique, soit par filtration, soit par dialyse soit par chromatographie d'exclusion, ce qui élimine également le PBQ qui n'aurait pas réagi, soit par addition d'une base. Le pH est amené vers 8 à 11,5, dépendant de la constante de dissociation des groupements réactionels de l'haptène destiné au couplage, pour ensuite procéder au couplage des haptènes, soit par leur fonction -NH₂ (pH de couplage compris préférentiellement entre 9,5 et 12) soit par la fonction -SH des haptènes sulfhydrylés (pH de couplage compris préférentiellement entre 8 et 10,5).

Les haptènes sulfhydrylés sont préférés. Les haptènes sulfhydrylés sont introduits dans la solution de polymère activé dans une proportion variable selon le couplage désiré, en fonction du poids moléculaire de l'haptène sulfhydrylé ou de son dérivé, par rapport au nombre de molécules de PBQ introduites sur la protéine. Un excès de molécules d'haptène est souhaitable par rapport au nombre de molécules d'agent couplant, préférablement le PBQ, introduites. Normalement, le poids de l'haptène excède 4 à 5 fois le poids de PBQ introduit. La réaction de couplage est pratiquement instantanée.

L'excès de molécules hapténiques est éliminé par dialyse, chromatographie ou filtration et le conjugué est stocké à -20 ^{o}C jusqu'à son utilisation pour développer des trousses diagnostiques, pour développer un système d'isolation d'anticorps spécifiques par chromatographie d'affinité ou encore pour développer des vaccins.

### 1. Synthèse de NO-cystéine et de NO-N-acétyl-L-cystéine.

La cystéine et la N-acétyl-L-cystéine (NAC), couplées ou non à la protéine porteuse, sont associés au -NO de la façon suivante : les produits sont acidifiés, normalement par 0,5 N ou 1 N HC1 (pour la cystéine), mais une solution de KH₂P0₄ ou une solution d'acide succinique ou tout autre acide, peuvent également convenir pour la NAC, qui n'est pas précipitée par ces acides. Le nitrite de sodium (NaN0₂) est pesé et dissous dans la solution contenant les molécules possédant un groupement sulfhydryl. Normalement, un excès de facteur 3 à 4 de molécules de nitrite est ajouté aux molécules de cystéine ou de dérivés de cystéine porteurs d'un groupement sulfhydryl, présents dans la solution. La solution acide est placée à 37 ^{o}C durant au moins 30 minutes, préferentiellement 60 minutes, pour que la liaison avec le NO puisse avoir lieu. Le pH de la solution est ensuite ramené vers la neutralité par addition d'un tampon pH 7,0 Tris ou phosphate concentré. Les autres haptènes de l'invention sont nitrosylés selon une méthode identique à celle décrite pour la cystéine. Des molécules très réactionnelles, telles que le glutathion et l'hémoglobine, ne nécessitent pas d'amener le pH vers une acidité prononcée pour obtenir une réaction : la réaction de nitrosylation a lieu à pH neutre.

### 2.a. Sensibilisation d'une phase solide par le conjugué de l'invention.

Les puits de plaques de microtitration, préférentiellement NUNC maxisorb, sont couverts par une solution (100 µl / puits) de NaCl 0,9 % tamponnée à pH 7,2 contenant entre 2 et 40 µg de protéine conjuguée, préférentiellement de l'albumine conjuguée durant 24 heures à 4 ^{o}C, à température ambiante ou à 37 ^{o}C. Un tampon pH 9,6 est également adéquat, ou tout autre milieu de sensibilisation qui favorise l'adsorption de la protéine conjuguée sur le plastique des puits. Après 24 heures ou plus d'incubation, les puits sont lavés par une solution 0,9% de NaCl ou par toute autre solution de lavage, et ensuite stockés à 4 ^{o}C avant leur utilisation dans un test ELISA de détection d'anticorps anti-haptènes.

### 2.b. Sensibilisation d'une phase solide par les haptènes de l'invention.

Préférentiellement, une phase solide constituée de polystyrène (plaques de microtitration) activée par une maléimide est utilisée. Chaque puits de la plaque de microtitration activée par la maléimide est incubée avec 0,1 à 2 µg d'un haptène sulfhydrylé selon l'invention, contenu dans 100 à 200 µl d'une solution minérale aqueuse tamponnée à pH légèrement acide (pH 6 à pH 6,8). Après une heure d'incubation à température ambiante, les puits sont lavés. Les fonctions maléimides subsistantes sont inactivées par incubation des puits avec une solution à 0,2% de poudre de lait écrémé durant une heure à température ambiante.

### 3. Chromatographie d'affinité.

Du sépharose activé par du CNBr ou par tout autre agent couplant est disponible commercialement (Pharmacia, Uppsala, Suède). Le procédé de couplage de protéines porteuses telles que de l'albumine à cette suspension de gel de sépharose activé est connue de l'Homme de l'Art : le gel est lavé par une solution acide sur un filtre en céramique ou verre fritté, le pH de la solution de suspension du gel est basifié, généralement amené à pH 7,2 ou 8. La protéine porteuse d'haptènes destinée au couplage est mélangée au gel et la réaction de couplage a lieu durant 24 heures à température ambiante. Le gel est ensuite lavé et utilisé pour l'adsorption d'anticorps anti-haptènes ou antigènes fixés de façon covalente sur le gel par l'intermédiaire de la protéine porteuse. La réaction immunologique d'adsorption des anticorps sur les haptènes liés au gel se fait soit en batch soit sur une colonne, à pH neutre en milieu normalement 0,9 % NaCl. L'élution des anticorps spécifiques s'effectue soit par des chaotropes, soit par des solutions acides (glycine-HCl pH 2) soit par de l'eau distillée basifiée par NH₄0H à pH 12. Ces méthodes sont toutes bien connues de l'Homme de l'Art.

### 4. Vaccination.

Des oiseaux et des mammifères sont vaccinés par une solution d'albumine originaire de la même espèce, à laquelle a été couplé un haptène. La solution vaccinante peut être complétée par des adjuvants qui amplifieront la réaction immunologique de production d'anticorps induite par le vaccin.

### 5. Détection d'anticorps de classe IgG et de classe IgM anti-N-acétyl-L-cystéine.

100 mg d'albumine bovine délipidée ont été mis en solution dans 5 ml d'eau contenant 5 mg de KH₂P0₄. 5 mg de PBQ ont été mis en solution dans 1 ml de méthanol, dont 0,25 ml (1,25 mg) ont été ajoutés à la solution de protéine. Après 1 heure à température ambiante et à l'obscurité, 2 ml de la solution (40 mg ) ont été dilués par 2 ml d'une solution contenant 4 mg / ml de KH₂P0₄.3H20 et 2,5 mg de N-acétyl-L-cystéine Après 30 minutes d'attente, la solution d'immunoconjugué a été amenée à pH 7,2 par addition d'un tampon phosphate concentré. Une deuxième préparation d'immunoconjugué, constituée également de 4 ml contenant 40 mg de protéine, a été complétée par 12,5 mg de nitrite de soude et par 13 mg d'acide succinique. Après dissolution des ingrédients ajoutés, la solution d'immunoconjugué a été placée à 37 ^{o}C durant 1 heure. La solution a été ensuite ramenée vers la neutralité par addition de 110 mg de KH₂P0₄.3H20. Une solution d'albumine traitée de façon identique mais avec de la glutamine au lieu d'acétyl-cystéine, a également été préparée. Une solution d'albumine sans haptène traitée de la même façon que les immunoconjugués a également été préparée.

Les immunoconjugués ont servi à fabriquer des plaques de microtitration pour la détermination de la présence d'anticorps dans un échantillon : 20 µg d'immunoconjugué par ml de solution de sensibilisation ont été préparés dans un milieu de sensibilisation constitué de NaCl 0,9%, amené à pH 7,2 par un tampon phosphate 0,05 M. Après 18 heures d'incubation dans des puits de plaques de microtitration Maxisorb de Nunc, les puits ont été lavés par 0,9 % NaCl puis incubés durant 60 minutes à 37 ^{o}C par 100 µl de divers sérums humains dilués au centième dans une solution NaCl 0,9 % pH 7,2 contenant 0,2 % de tween 20. Après lavage par une solution tamponnée de NaCl contenant 0,1% de tween, les puits ont été recouverts durant 30 minutes par trois anticorps anti-IgG, IgA et IgM humains, marqués à la peroxydase. Après lavage, la présence éventuelle de peroxydase, proportionnelle à la présence d'anticorps spécifiques pour les haptènes ou pour l'albumine sensibilisant les puits, a été déterminée par une réaction enzymatique colorée basée sur le peroxyde d'hydrogène en présence de tétraméthylbenzidine. La réaction enzymatique produit une coloration bleue qui vire au jaune après addition d'une solution d'arrêt acide (0,5N H₂SO₄). L'absorbance obtenue est mesurée à 450 nm. Les résultats sont donnés dans le tableau 1 , pour cinq sérums numérotés de 1 à 5.

**Tableau 1**

| | Albumine | | | NO-N-acétyl-cystéine | | | N-acétyl-L-cystéine | | |
|---|---|---|---|---|---|---|---|---|---|
| | IgG | IgM | IgA | IgG | IgM | IgA | IgG | IgM | IgA |
| 1 | 0,3 | 0,31 | 0,56 | 0,30 | 0,28 | 0,55 | 0,30 | 0,28 | 0,45 |
| 2 | 0,57 | 0,42 | 0,46 | 0,49 | 0,31 | 0,44 | 0,36 | 0,30 | 0,44 |
| 3 | 0,78 | 0,33 | 0,45 | 0,77 | 0,31 | 0,40 | 0,70 | 0,30 | 0,40 |
| 4 | 0,30 | 0,23 | 0,47 | 1,51 | 0,25 | 0,46 | 1,44 | 0,24 | 0,48 |
| 5 | 0,29 | 0,48 | 0,34 | 0,27 | 1,42 | 0,33 | 0,24 | 1,33 | 0,31 |

Les résultats obtenus avec le conjugué glutamine étaient similaires au contrôle albumine et ne sont pas donnés ici, par souci de clarté. On voit que le serum 4 est positif en IgG pour la N-acétyl-L-cystéine (NAC), légèrement plus positif en présence de NAC-NO, tandis que le sérum 5 est positif en IgM, pour les mêmes haptènes.

### 6. Mesure de la spécificité de la réaction immunologique.

Une vérification de la spécificité de la réaction immunologique a été effectuée par une réaction de compétition. Le sérum 4, réagissant immunologiquement avec la NAC immobilisée mais pas avec l'albumine, a été mis en contact en solution avec le même haptène couplé à de l'albumine, en concentrations décroissantes. La concentration de l'immunoconjugué, basé sur le poids de l'albumine porteuse, a été variée de 100 µg / ml à zéro, par dilutions de moitié, puis diluées de moitié avec une solution contenant l'anticorps anti-NAC. Les mélanges réactionnels ont été placés à 37 ^{o}C. Après 30 minutes d'incubation à 37 ^{o}C, les différents échantillons de sérum adsorbé ont été analysés pour la présence d'anticorps anti-NAC résiduels. Les résultats sont assemblés dans le tableau 2.

**Tableau 2**

| Immunoconjugué (µg / ml) | Absorbance 450 nm |
|---|---|
| 50 | 0,42 |
| 25 | 0,68 |
| 12,5 | 0,98 |
| 6,75 | 1,22 |
| 3,375 | 1,42 |
| 1,65 | 1,55 |
| 0,825 | 1,60 |
| 0 | 1,75 |

On voit que l'incubation du sérum 4 en présence d'haptène durant 30 minutes à 37 ^{o}C avant l'analyse ELISA, réduit considérablement l'adsorbance obtenue, et ce de façon proportionelle à la concentration de l'immunoconjugué en solution, indiquant une inhibition spécifique de la réaction immunologique par l'NAC en solution.

### 7. Interférence immunologique due à la cystéine libre.

Une analyse de la spécificité de la réaction immunologique de l'anticorps IgG anti-NAC a été effectuée avec de la cystéine libre, en suivant le même protocole que dans l'exemple 2. Les résultats sont assemblés dans le tableau 3.

**Tableau 3**

| Cystéine libre (µg / ml) | Absorbance 450 nm |
|---|---|
| 10 | 1,51 |
| 5 | 1,59 |
| 2,5 | 1,65 |
| 1,25 | 1,68 |
| 0,625 | 1,72 |
| 0,312 | 1,73 |
| 0,156 | 1,76 |
| 0 | 1,77 |

On voit que le même phénomène d'inhibition de la réaction immunologique de l'anticorps avec l'haptène NAC couplé sur les plaques observé dans l'exemple 2 avec des serums incubés en présence d'NAC-albumine libre, s'observe également lorsque l'anticorps est incubé préalablement durant 30 minutes à 37 ^{o}C en présence de cystéine libre. La relation directe observée entre la dose de cysteine et l'inhibition provoquée plaide fortement en faveur d'une inhibition spécifique. L'inhibition observée est beaucoup moins prononcée que celle obtenue avec la NAC couplée à l'albumine, indiquant que les anticorps sont plus spécifiques pour la NAC mais présentent une interférence avec la cystéine. L'isolation et la purification de ces anticorps devraient permettre un traitement différent de sujets souffrant de syndrômes d'immunodéficience.

### 8. Analyse de la spécificité des anticorps IgG et IgM envers la cystéine et le -NO.

Il est connu que les gammaglobulines peuvent être dégradées par la cystéine à pH basique. Il est également connu que non seulement des agents réducteurs tels la cystéine mais également des oxydants dégradent les gammaglobulines. Il reste à exclure la possibilité que la cystéine et le NO interfèrent avec la réaction immunologique. Une analyse a été effectuée sur un sérum IgG et un serum IgM trouvés positifs dans un test ELISA pour la NAC-NO couplée à de l'albumine ainsi que sur un serum IgG positif pour un antigène tuberculeux. L'analyse sur un anticorps antituberculeux permet de vérifier de façon indépendante les possibles interférences dues à l'utilisation d'agents réducteurs (cystéine et NAC) et oxydants (NO). Le contrôle interne de la spécificité de la réaction immunologique a été réalisé en testant la réaction immunologique en présence de cystine (forme réduite de la cystéine) et de NaNO₂ activé à pH acide de la même façon que la cystéine et la (NAC), mais en l'absence de ces récepteurs. Une deuxième expérience a été ensuite effectuée pour confirmer et complèter les observations interessantes acquises lors de la première analyse. Les deux analyses ont été conduites comme dans les exemples 2 et 3, c'est-à-dire que des haptènes libres présents dans une solution à des concentrations décroissantes ont été mis en contact avec des sérums trouvés positifs (en IgG ou en IgM) dans un test sérologique ELISA pour l'haptène NAC ou pour un antigène tuberculeux. Les haptènes compétitifs mis en contact en solution avec les anticorps IgG et IgM anti-N-acétyl-cystéine et IgG anti-tuberculeux étaient la cystine, le NaNO₂ acidifié puis ramené à pH neutre, la cystéine, la cystéine-NO, l'acétyl-cystéine, l'acétyl-cystéine-NO, et l'acétyl-cystéine-NO couplé à de l'albumine. Après 30 minutes d'incubation à 37 ^{o}C, les solutions contenant les haptènes libres et les anticorps ont été analysés pour la présence résiduelle d'anticorps anti-NAC ou anti-tuberculeux. Les résultats de ces deux analyses sont donnés dans le tableau 4.

**Tableau 4**

| Concentration finale (µg/ml) en haptènes et en NAC-albumine compétitifs libres | | | | | |
|---|---|---|---|---|---|
| | 50 | 25 | 12,5 | 6 | 0 |
| | | | | | |

| **Sérum positif IgG** 1^{ère} expérience | | | | | |
|---|---|---|---|---|---|
| L-cystine | 0,90 | 0,88 | 0,83 | 0,87 | 0,84 |
| HNO₂ | 0,77 | 0,85 | 0,84 | 0,86 | 0,84 |
| L-cystéine | 0,65 | 0,68 | 0,70 | 0,84 | 0,84 |
| L-cystéine-NO | 0,84 | 0,84 | 0,85 | 0,86 | 0,84 |
| NAC | 0,27 | 0,24 | 0,27 | 0,38 | 0,84 |
| NAC-albumine | 0,36 | 0,40 | 0,69 | 0,65 | 0,84 |

| 2^{ème} expérience | | | | | |
|---|---|---|---|---|---|
| NAC | 0,23 | | 0,38 | | 1,15 |
| NAC-NO | 0,22 | | 0,37 | | 1,07 |
| Cystéine | 0,81 | | 1,12 | | 1,29 |
| Cystéine-NO | 1,22 | | 1,21 | | 1,29 |

| **Sérum positif IgM** 1^{ère} expérience | | | | | |
|---|---|---|---|---|---|
| L-cystine | 1,49 | 1,52 | - | 1,44 | 1,61 |
| HNO₂ | 1,55 | 1,45 | 1,49 | 1,51 | 1,50 |
| L-cystéine | 1,45 | 1,43 | 1,42 | - | 1,37 |
| L-cystéine-NO | 1,54 | 1,55 | 1,50 | 1,48 | 1,59 |
| NAC | 1,46 | 1,44 | 1,40 | 1,52 | 1,52 |
| NAC-albumine | 1,45 | 1,47 | 1,50 | 1,55 | 1,50 |

| 2^{ème} expérience | | | | | |
|---|---|---|---|---|---|
| NAC | 1,61 | | 1,76 | | 1,88 |
| NAC-NO | 1,51 | | 1,77 | | 1,89 |
| Cystéine | 1,53 | | 1,58 | | 1,84 |
| Cystéine-NO | 1,70 | | 1,74 | | 1,83 |

| **Sérum positif IgG TUB** | | | | | |
|---|---|---|---|---|---|
| L-cystine | 1,12 | | | | 1,07 |
| HNO₂ | 1,04 | | | | 1,04 |
| L-cystéine | 1,07 | | | | 1,02 |
| L-cystéine-NO | 1,07 | | | | 1,02 |
| NAC | 1,11 | | | | 1,00 |
| NAC-albumine | 1,06 | | | | 1,00 |

Cette analyse apporte plusieurs informations, entre autres : ni la cystine, ni le HNO₂ dans les conditions d'applications de l'analyse immunologique n'influent sur le résultat. Le NAC couplé à l'albumine est un inhibiteur efficace, compte tenu de sa concentration réduite, par rapport à la molécule NAC utilisée libre. L'anticorps IgG est plus susceptible à l'inhibition que l'anticorps IgM. L'inhibition provoquée par la molécule NAC libre est plus efficace que celle due à la molécule cystéine libre. Le NO réduit l'inhibition due à la cystéine mais n'atteint pas celle due à la NAC. Au contraire, il semble s'amplifier.

### 9. Analyse des IgM d'une population de malades souffrant de sclérose en plaques (SEP).

Une analyse des IgM de personnes ne souffrant pas de maladies (tests de grossesse, accidents de la route, etc) versus malades souffrant de sclérose en plaque est donnée dans la figure l. Les absorbances observées pour la NAC sont normalisées versus les adsorbances observées dans la trousse contrôle (albumine-glutamine). On voit qu'un seul sujet sain possède des anticorps légèrement au dessus de la norme, que la moyenne (0,3) obtenue pour la population saine est significativement différente de la moyenne établie chez les SEP (0,5), avec un malade spectaculairement positif.

### 10. Analyse des IgG d'une population de séropositifs pour le HIV.

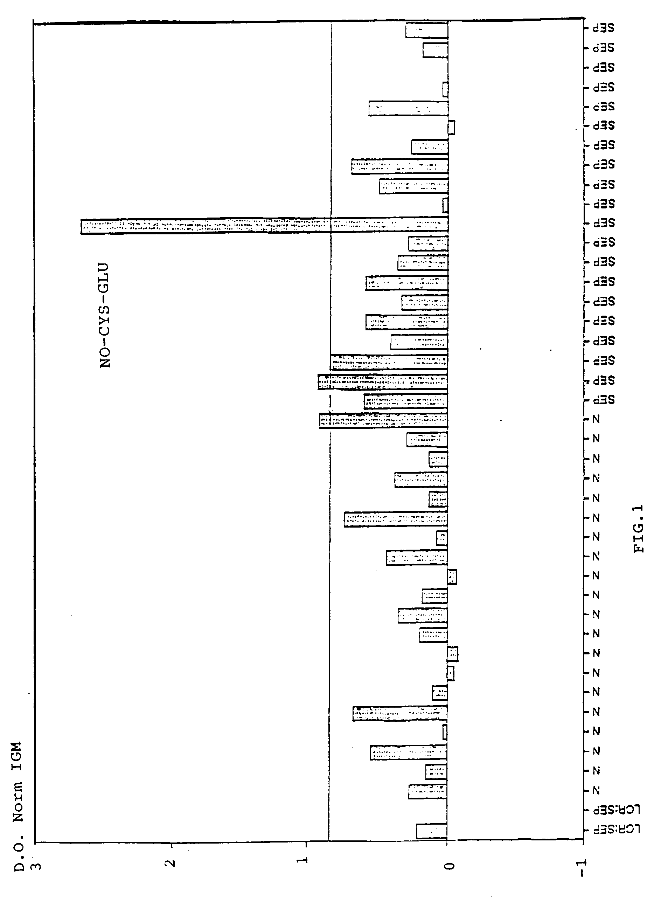

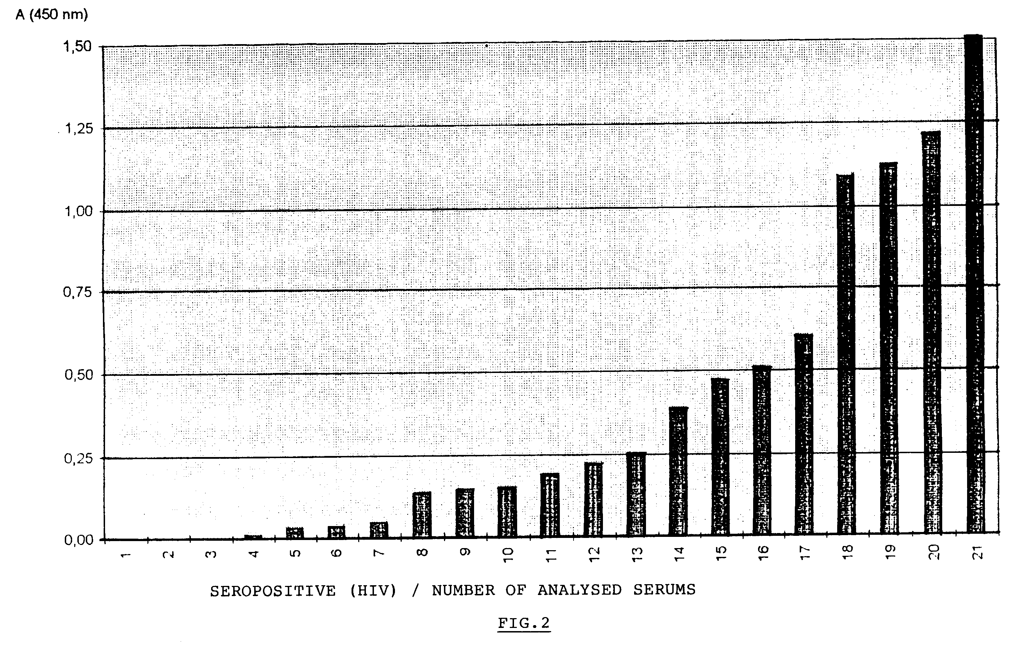

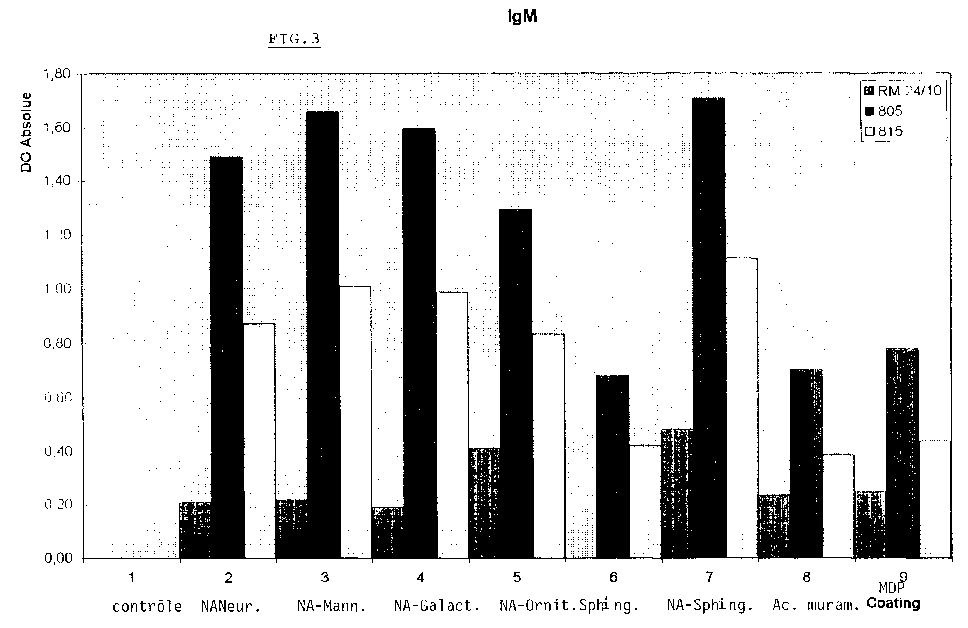

Une analyse des IgG spécifiques de l'acétylcystéine de personnes séropositives pour le virus HIV I est donnée dans la figure 2. On voit que plusieurs sujets sont fortement positifs pour l'acétylcystéine.

### 11. Interférence des anticorps anti-acétylcystéine avec le glutathion et avec le gutathion nitrosylé.

Le glutathion a été nitrosylé de la même façon que la cystéine et l'acétylcystéine, comme décrit supra. Sept sérums riches en anticorps anti-acétyl-cystéine ont été adsorbés durant 30 minutes à 37 ^{o}C avec 500 nanomoles d'acétyl-cystéine libre, de glutathion libre ou de glutathion nitrosylé, libre. Les sérums adsorbés ont été ensuite analyses pour la présence d'anticorps de classe IgG spécifiques pour l'acétyl-cystéine. Le pourcentage d'activité résiduelle est donné dans le tableau 5. On voit que la nitrosylation du glutathion amplifie son pouvoir de compétition avec les anticorps. Le sérum 7 est inhibé de façon similaire par l'acétylcystéine libre (9% d'activité sérologique résiduelle) et par le glutathion nitrosylé libre (5% d'activité sérologique résiduelle).

**Tableau 5**

| **analyse de l'interférence de 7 sérums positifs** | | | |
|---|---|---|---|
| **Pourcentage d'adsorbance résiduelle** | | | |
| **Sérum** | **NAC** | **Glut.** | **Glut.-NO** |
| 1 | 12.7 | 82.5 | 42.9 |
| 2 | 18.3 | 100.0 | 71.4 |
| 3 | 2.7 | 69.3 | 57.3 |
| 4 | 21.8 | 85.0 | 63.2 |
| 5 | 11.1 | 79.0 | 65.4 |
| 6 | 7.0 | 56.1 | 31.6 |
| 7 | 9.0 | 66.7 | 5.0 |

### 12. Analyse IgG et IgM de sérums provenant de patients souffrant d'afflictions diverses, avec des immunoconjugués préparés selon l'invention.

Les résultats obtenus avec la cystéine, l'acétyl-cystéine, le glutathion et leurs dérivés nitrosylés ont montré de façon tout à fait inattendue que des anticorps humains contre un monopeptide (c'est-à-dire un peptide constitué d'un seul acide aminé), contre un dérivé de cet acide aminé, et a fortiori contre un tripeptide comprenant l'acide aminé immunogène, existaient de façon naturelle et pouvaient être mis en évidence par les conjugués de l'invention. Il restait à montrer que l'existence de tels anticorps contre un acide aminé n'était pas restreinte à cet acide aminé particulier, mais pouvait être détectée dans le cas d'autres molécules de poids moleculaire faible situé entre 200 et 400 KD, que des molécules de poids moléculaire faible telles que les carbohydrates étaient également immunogéniques dans certaines conditions pathologiques, et finalement que ces anticorps à l'existence insoupçonnée jusqu'à présent pouvaient être mis en évidence par un système de détection suffisamment puissant.

Des immunoconjugués produits à partir de mercaptoéthanol, de dérivés d'acides aminés et de carbohydrates ont été préparés par activation d'une protéine porteuse au moyen de PBQ et substitution du résidu aminé des haptènes par le réactif de Traut, au pH modérément basique de 9,0. Les carbohydrates non-aminés ont été substitués à pH 11.

Le nombre de conjugués susceptibles d'être synthétisés par la présente méthode et le nombre de molécules susceptibles d'être analysées est extrêmement élevé. Pour montrer l'universalité de la méthode, les Inventeurs ont essentiellement choisi des molécules de poids moléculaire faible (dérivés d'acides aminés et de carbohydrates), sans s'efforcer de démontrer que des molécules de poids moléculaire plus élevé, c'est-à-dire des dipeptides et des dissaccharides ainsi que des polypeptides et des oligosaccharides, sont elles aussi imunogènes : une antigénéicité démontrée pour un monopeptide et/ou dérivé d'acide aminé et pour un monosaccharide et/ou dérivé de monosaccharide s'étendra le plus souvent aussi à la même molécule incluse dans une formation de poids moléculaire plus élevé comme il a été montré avec la cystéine et le glutathion.

Les molécules suivantes ont été analysées dans leurs fonction antigénique : la N-acétyl-cystéine, la N-acétyl-cystéine-NO, la N-acétyl-L-tryptophane, la N-acétyl-L-tryptophane-NO, la tryptamine, la sérotonine, la sérotonine-NO, la 5-méthoxytryptamine, la glycine, la L-glutamine, la L-arginine, la L-arginine-β-naphtylamide, la N-acétyl-arginine, la N-acétyl-arginine-NO, la N-acétyl-sérotonine, le tryptophane, la L-valine-β-naphtylamide, la L-valine, la N-valine, le L-tryptophane-β-naphtylamide, la L-alanine-β-naphtylamide, l'acétyl-phénylalanine-β-naphtylamide, la cystéine-glycine, la N-acétyl-glucosamine, la N-acétyl-glucosamine-NO, le mercapto-éthanol, le mercapto-éthanol-NO, la N-acétyl-ornithine, la N-acétyl-mannosamine, la N-acétyl-sphingosine, la D-sphingosine, l'acide N-acétyl-neuraminique, l'acide N-acétyl-D-galactosamine, l'acide N-acétyl-muramique, l'acide muramique, la N-acétyl-muramyl-L-alanine-D-isoglutamine (MDP), phosphatidylinositol, ... .

La sphingosine et l'acétylsphingosine sont insolubles en milieu aqueux et ont été substituées de la façon suivante : 1 mg de sphingosine et 1,5 mg d'acétyl-sphingosine ont été solubilisés dans 0,1 ml et 0,2 ml de méthanol sec. 420 µg de réactif de Traut solubilisés dans 28 µl de méthanol anhydre ont été ajoutés à la solution d'acétyl-sphingosine. La réaction de substitution a eu lieu durant 45 minutes à 37 ^{o}C. Après ce temps, une solution de 10 mg d'albumine bovine délipidée activée par 100 µg de PBQ à pH 6.0 a été amenée à pH 8 et ajoutée à la solution organique, rapidement en une seule fois. Le mercapto-éthanol a été couplé directement par son résidu thiol à l'albumine activée par le PBQ. Certains immunoconjugués ont été également nitrosylés selon la technique décrite. Des sérums provenant de malades atteints de sclérose en plaques, de lupus, de syndrome d'immuno-déficience acquis (AIDS), de cirrhose, de myasthénie, de vascularite, de cancer, de migraine, de leucémie, de sclérose latérale, de Wallenstroem, d'arthrite et polyarthrite, de polymyosie, de rhumatisme articuliare, de lymphome, d'infarctus, d'accident vasculaire cérébral et de tuberculose ont été systématiquement analysés pour la présence d'anticorps de classe IgG et de classe IgM contre ces haptènes.

Parmi les conjugués étudiés, il ressort que les conjugués de N-acétyl-arginine nitrosyle, de N-acétyl-5-hydroxytryptamine nitrosylé et de N-acétyl-glucosamine sont fréquemment positifs pour certaines afflictions. Des patients atteints de sclérose en plaques, de lupus, de cirrhose, de myasthénie, de tuberculose montrent fréquemment des positivités élevées tandis que les patients atteints d'autres maladies (infections diverses, vascularite, Parkinson, dermatopolymyosite) ne semblent pas posséder de composante auto-immune détectable par les immuno-conjugués mis en oeuvre.

Les dérivés portant la fonction β-naphtylamide n'ont été trouvés positifs dans aucun cas. La N-acétyl-ornithine est fréquemment élevée chez les patients atteints du SIDA et de sclérose en plaques, tandis que des titres élevés d'anticorps contre l'acétyl-sphingosine sont absents chez les sidéens mais se retrouvent chez les malades atteints de sclérose en plaques et les migraineux.

Le tableau 6 ci-dessous compare les données IgG obtenues en utilisant la N-acétyl-mannosamine et la sphingosine couplées à de l'albumine pour identifier la présence éventuelle d'anticorps chez des migraineux et des tuberculeux.

**Tableau 6**

| **N° sérum** | **Migraine** | | **Tuberculose** | |
|---|---|---|---|---|
| | **N-A-mann.NH**_{**2**} | **Sphingosine** | **N-A-mann.NH**_{**2**} | **Sphingosine** |
| **1** | 0,11 | 0,71 | 0,41 | 0,15 |
| **2** | 0,00 | 0,77 | 0,47 | 0,10 |
| **3** | 0,00 | 0,62 | 0,17 | 0,08 |
| **4** | 0,00 | 0,38 | 0,19 | 0,18 |
| **5** | 0,00 | 0,20 | 0,22 | 0,12 |
| **6** | 0,00 | 0,00 | 0,24 | 0,31 |
| **7** | 0,00 | 0,00 | 0,28 | 0,22 |
| **8** | 0,20 | 0,00 | 0,31 | 0,08 |
| **9** | 0,10 | 0,00 | 0,32 | 0,18 |
| **10** | 0,46 | 0,32 | 0,28 | 0,08 |

On voit que les migraineux n'ont pas d'anticorps anti-N-acétyl-mannosamine, mais que certains d'entre eux ont des anticorps anti-sphingosine et l'inverse est observé chez les tuberculeux.

Une analyse de la présence d'anticorps anti-mercapto-éthanol-NO dans 8 groupes de malades souffrant d'afflictions diverses montre que les malades souffrant de sclérose en plaques n'ont pas d'anticorps de type IgG réagissant avec cet haptène. Les malades affectés par le SIDA sont très faiblement positifs. La fréquence de positivité et l'intensité de la réaction augmentent dans d'autres groupes : les malades dépressifs et ceux souffrant de la maladie de Kahler (myelome) sont très fortement réactionnels (voir tableau 7).

**Tableau 7**

| SEP | SIDA | Migraine | Tuberc. | Asthme | Crohn | Dépression | Myelome |
|---|---|---|---|---|---|---|---|
| 0.26 | 0.21 | 0.41 | 0.13 | 0.45 | 0.49 | 1.44 | 1.09 |
| 0.25 | 0.26 | 0.50 | 0.14 | 0.39 | 1.09 | 1.61 | 1.49 |
| 0.26 | 0.34 | 0.47 | 0.21 | 0.31 | 1.37 | 1.47 | 1.85 |
| 0.18 | 0.35 | 0.53 | 0.19 | 0.49 | | 1.12 | 1.53 |
| 0.21 | 0.35 | 0.47 | 0.18 | 0.89 | | | 1.79 |
| 0.17 | 0.33 | 0.48 | 0.36 | 1.20 | | | 1.31 |
| 0.24 | 0.33 | 0.36 | 0.45 | 1.27 | | | 0.79 |
| 0.16 | 0.30 | 0.09 | 0.36 | 1.24 | | | 0.66 |
| 0.18 | 0.38 | 0.23 | 0.47 | 1.01 | | | 0.73 |
| 0.14 | 0.55 | 0.15 | 0.43 | 0.80 | | | 0.92 |

14 séra de malades souffrant de polyarthrite rhumatoïde ont été analysés pour la présence d'anticorps anti-carbohydrates. 7 séra présentaient des taux d'anticorps élevés, de classe soit IgG, soit IgM, contre l'acide N-acétyl-neuraminique, la N-acétyl-mannosamine et la N-acétyl-galactosamine. Les réponses immunologiques les plus fortes ont été observées contre la N-acétyl-galactosamine. Cette observation est à mettre en relation avec l'observation de taux assez élevés d'anticorps de même nature chez deux tuberculeux avancés, versus un témoin sain très récemment vacciné. Les résultats d'une analyse IgM pour la présence d'anticorps contre l'acide N-acétyl-neuraminique (colonne 2), la N-acétyl-mannosamine (colonne 3), la N-acétyl-galactosamine (colonne 4), la N-acétyl-ornithine (colonne 5), la sphingosine (colonne 6), la N-acétyl-sphingosine (colonne7), l'acide muramique (colonne 8) et le muramyl-dipeptide (colonne 9) sont donnés dans le tableau 8, après soustraction des valeurs contrôles (albumine seule). On voit l'importance des réponses observées contre les carbohydrates et le N-acétyl-sphingosine.

### 13. Analyse comparée de présence d'anticorps anti-N-acétyl-cystéine sur des plaques de microtitration sensibilisées par un conjugué ou par l'haptène.

Des plaques de microtitration NUNC Maxisorb ont été sensibilisées par un conjugué composé de N-acétyl-cystéine couplé à de l'albumine bovine, comme déjà décrit plus haut. Le même haptène a été utilisé pour sensibiliser les cupules de plaques de microtitration COSTAR-"sulhydryl binding surface ®" (cat. 2509-K0419143). 100 µl d'une solution à 20 µg/ml d'N-acétyl-cystéine solubilisée dans un tampon phosphate 50 mM à pH 6,5 ont été introduits dans les cupules activées par la maléimide et incubés durant 60 min. à température ambiante. Après sensibilisation, les cupules ont été vidées et lavées 3 fois par une solution 0,15M NaCl et les cupules ont été ensuite saturées par une solution 0,2% de poudre de lait écrémé durant 30 min. à température ambiante. Les malades du SIDA ont été analysés pour la présence d'anticorps IgM anti-N-acétyl-cystéine, comme dans l'exemple 10. Le test pratiqué avec des cupules sensibilisées par l'hapatène seul sans protéine porteuse est nettement moins sensible, avec nécessité d'appliquer des dilutions sériques 1:25 pour obtenir un signal avec un bruit de fond important observé avec des séra privés d'anticorps spécifiques anti-NAC.

## Revendications

1. Conjugué immunoréactif comprenant un ou plusieurs haptène(s) comportant un groupe sulfhydryl et choisi(s) parmi le groupe constitué par les acides aminés, les carbohydrates, les carbohydrates aminés, le phosphatidylinositol, la sphingosine et leurs dérivés nitrosylés, acylés et/ou acétylés, ledit haptène étant couplé à une protéine porteuse hydrosoluble d'un poids moléculaire supérieur à 8000 KD et/ou un support solide par un agent couplant susceptible de se fixer au groupement sulfhydryl dudit haptène.

2. Conjugué immunoréactif selon la revendication 1, caractérisé en ce que l'haptène est choisi parmi le groupe constitué par la cystéine, la N-acétyl-L-cystéine, la cystéine-NO, la N-acétyl-L-cystéine-NO, la L-γ-glutamyl-L-cystéine, la N-acétyl-cystéine-glycine, le glutathion, l'homocystéine, le mercapto-éthanol, la méthionine, la thiolhistidine, l'acétylméthionine et la S-adénosylhomocystéine.

3. Conjugué immunoréactif selon la revendication 1, caractérisé en ce que l'haptène est une molécule dont les groupements amines et/ou hydroxyl ont été substitués par un groupement sulfhydryl.

4. Conjugué immunoréactif selon la revendication 3, caractérisé en ce que la molécule est choisie parmi le groupe constitué par le phosphatidylinositol, la glutamine, l'arginine, l'ornithine, l'homosérine, le diéthylnitrosamine, la carnitine, le N-éthyl-N-nitrosourée, le N(ω)-nitro-L-arginine-méthyl-ester, la taurine, la nicotine, la N^{α}-acétyl-lysine, la N^{ε}-(carboxyméthyl) lysine, la N^{ε}-(carboxyméthyl) hydroxylysine, la N^{α}-acétyl-N^{ε}-(carboxyméthyl) lysine, les indolamines, en particulier le tryptophane, le 5-méthoxytryptophane, le N-acétyl-tryptophane et le tryptophane acylé, la 5-hydroxytryptamine (sérotonine), la 6-hydroxytryptamine, la 5-méthoxytryptamine, la 5,6-dihydroxytryptamine, l'acide 5-méthoxyindolacétique, l'acide 5-hydroxyindolacétique, la N-acétyl-5-méthoxytryptamine (mélatonine), le 5-méthoxytryptophol, le 5-hydroxyptryptophol, les dérivés de sphingosine, en particulier la D-sphingosine, la sphingosyl-galactopyranoside, la sphingosyl-phosphoryl-choline et la N-acétyl-D-sphingosine, les carbohydrates tels que le glucose, le mannose, le galactose, l'arabinose, l'acide muramique, leurs dérivés aminés tels que la glucosamine, la galactosamine, la mannosamine, le -α-(1→5) D-arabinofuranoside ou encore le 3,6-di-O-Me-β-D glucopyranosyl-(1→4)-2,3-di-O-Me-α-L-rhamnopyranose, le trisaccharide L-α-D-heptose (1→2) heptose (1→3) heptose, leurs dérivés acétylés, en particulier l'acide N-acétyl-neuraminique, l'acide N-acétyl-O-neuraminique, la N-acétyl-glucosamine, la N-acétyl-mannosamine, la N-acétyl-galactosamine, l'acide N-acétyl-muramique, la N,N-diacétyl-galactosamine, la N,N-diacétyl-glucosamine, leurs dérivés acylés, en particulier le stéarate, le palmitate, le phtiocéranate, l'hydroxyphtiocéranate, la N-acétyl-L-glutamine et la glutamine acylée, l'acide N-acétyl-glutamique et l'acide glutamique acylé, la N-acétyl-arginine et l'arginine acylée, la N-acétyl-ornithine et l'ornithine acylée, la N-acétyl-L-homosérine et l'homosérine acylée, le phosphatidyl-inositol mannoside (1-phosphatidyl-L-myo-inositol 2-O-α-D-mannopyranoside), le N-acétyl-muramyl-L-alanyl-D-isoglutamine et leurs dérivés nitrosylés.

5. Conjugué immunoréactif selon l'une quelconque des revendications précédentes, caractérisé en ce que la protéine porteuse est choisie parmi le groupe constitué par la gélatine, l'histone, l'albumine, de préférence l'albumine bovine délipidée, la polysérine ou la thyroglobuline.

6. Conjugué immunoréactif selon l'une quelconque des revendications précédentes, caractérisé en ce que le support solide est choisi parmi le groupe constitué par des globules rouges, du latex et/ou un support polymérique, de préférence des plaques de microtitration en polystyrène.

7. Conjugué immunoréactif selon l'une quelconque des revendications précédentes, caractérisé en ce que l'agent couplant est choisi parmi le groupe constitué par la parabenzoquinone, la p-toluquinone, la dichloroquinone, la chinhydrone, la 2,6-dichlorobenzoquinone, le N,N'-O-phénylènedimaléimide, le N-succinimidyl-3-(2-pyridyldithio)proprionate et le succinimidyl-4-(N-maléimidométhyl)cyclohexane-1-carboxylate.

8. Anticorps et/ou des fragments de celui-ci dirigé contre le conjugué immunoréactif selon l'une quelconque des revendications précédentes.

9. Anticorps et/ou des fragments de celui-ci dirigé contre l'anticorps et/ou des fragments de celui-ci selon la revendication 8.

10. Dispositif de diagnostic comprenant le conjugué selon l'une quelconque des revendications 1 à 7 et/ou un anticorps et/ou des fragments de celui-ci selon la revendication 8 et/ou 9.

11. Dispositif de diagnostic selon la revendication 10, caractérisé en ce qu'il est une trousse de diagnostic ou une colonne de chromatographie.

12. Composition pharmaceutique comprenant le conjugué selon l'une quelconque des revendications 1 à 7 et/ou un anticorps et/ou des fragments de celui-ci selon la revendication 8 et/ou 9 et éventuellement un véhicule pharmaceutique adéquat.

13. Procédé d'obtention du conjugué immunoréactif selon l'une quelconque des revendications 1 à 7, dans lequel un ou plusieurs haptène(s) comportant un groupe sulfhydryl et choisi(s) parmi le groupe constitué par les acides aminés, les carbohydrates, les carbohydrates aminés, le phosphatidylinositol, la sphingosine et leurs dérivés nitrosylés, acylés et/ou acétylés sont liés à une protéine porteuse hydrosoluble d'un poids moléculaire supérieur à 8000 KD et/ou un support solide par un agent couplant susceptible de se fixer au groupement sulfhydryl dudit haptène.

14. Procédé de détection et/ou de quantification d'anticorps spécifiques d'une pathologie comportant une réponse auto-immune, présent dans un fluide corporel d'un patient, caractérisé en ce que l'on prélève le fluide corporel du patient, en ce que l'on fait réagir les anticorps présents dans ce fluide corporel avec le conjugué immunoréactif selon les revendications 1 à 7 et en ce que l'on détecte et/ou quantifie la réaction croisée entre lesdits anticorps et le conjugué.

15. Utilisation de la composition pharmaceutique selon la revendication 12 pour la préparation d'un médicament destiné à la prévention et/ou au traitement de pathologies comportant une réponse auto-immune.

16. Utilisation selon la revendication 15, caractérisée en ce que les pathologies comportant une réponse auto-immune sont choisies parmi le groupe constitué par la sclérose en plaques, la sclérose amyotrophique latérale, l'hyperthyroïdie, l'arthrite, la cirrhose, les lupus, les syndrômes antiphosphatides, les syndrômes néphrotiques, la myasthénie, le cancer, la tuberculose et le syndrôme d'immuno-déficience acquis.
